# EUROPÄISCHE PATENTANMELDUNG

(11) **EP 3 178 378 A1**
(43) Veröffentlichungstag der Anmeldung: **14.06.2017**
(21) Anmeldenummer: 15199683.2
(22) Anmeldetag: 11.12.2015
(51) Int. Cl.: A61B 5/00, A61B 5/0408

(54) **VORRICHTUNG ZUR MESSUNG DER ELEKTRISCHEN HERZAKTION EINES TIERES**

(71) Anmelder: Tracer Systems GmbH, 8510 Stainz (AT)
(72) Erfinder: Klepp, Nikolaus, 4810 Gmunden (AT); Eichenauer, Heinz, 8524 Bad Gans (AT)
(74) Vertreter: Jell, Friedrich

(57) **Zusammenfassung**

Es wird eine Vorrichtung (1) zur Messung der elektrischen Herzaktion eines Tieres gezeigt, mit einem Gurt (2) und mit zumindest zwei am Gurt (2) vorgesehenen Elektroden (3), die je eine in Querrichtung (4) des Gurts (2) konvex gekrümmte Kontaktfläche (5) aufweisen. Um eine Vorrichtung zur Messung der elektrischen Herzaktion eines Tieres standfester zu gestalten und deren Tragekomfort zu erhöhen, wird vorgeschlagen, dass die konvex gekrümmten Kontaktflächen (5) mehrere Krümmungsabschnitte (6, 7) aufweisen, von denen die Krümmung des ersten Krümmungsabschnitts (7), der den Scheitelbereich der Kontaktfläche (5) ausbildet, geringer als die Krümmung eines an diesen ersten Krümmungsabschnitt (7) anschließenden zweiten Krümmungsabschnitts (6) ist.

## Beschreibung

Die Erfindung betrifft eine Vorrichtung zur Messung der elektrischen Herzaktion eines Tieres mit einem Gurt und mit zumindest zwei am Gurt vorgesehenen Elektroden, die je eine in Querrichtung des Gurts konvex gekrümmte Kontaktfläche aufweisen.

Um die Herzfrequenz bzw. der Herzratenvariabilität eines behaarten Tiers messen zu können, ist aus dem Stand der Technik eine als Gurt ausgebildete Vorrichtung bekannt (DE202012100735U1), deren durch Unterlegematerial aufgebrachte Elektroden in Querrichtung des Gurts konvex gekrümmte Kontaktflächen ausbilden. Nachteilig führen derart geformte Kontaktflächen mit steigender Tragedauer zur Bildung von Druckstellen und/oder zu Verletzungen am Tier, wodurch sowohl dessen Leistungsfähigkeit als auch die Verwendungsdauer des Gurts eingeschränkt werden. Zudem können derartige Gurte aufgrund eines schmerzbedingten Tragestresses für das Tier die Messergebnisse verfälschen und in weiterer Folge deren Messgenauigkeit beeinträchtigt.

Die Erfindung hat sich daher die Aufgabe gestellt, eine aus dem Stand der Technik bekannte Vorrichtung zur Messung der elektrischen Herzaktion eines Tieres dahingehend konstruktiv zu verbessern, dass mit dieser trotz einfacher Handhabung standfest genaue Messergebnisse aufgenommen werden können. Außerdem soll die Vorrichtung einen hohen Tragekomfort aufweisen.

Die Erfindung löst die gestellte Aufgabe dadurch, dass die konvex gekrümmten Kontaktflächen mehrere Krümmungsabschnitte aufweisen, von denen die Krümmung des ersten Krümmungsabschnitts, der den Scheitelbereich der Kontaktfläche ausbildet, geringer als die Krümmung eines an diesen ersten Krümmungsabschnitt anschließenden zweiten Krümmungsabschnitts ist.

Weisen die konvex gekrümmten Kontaktflächen mehrere Krümmungsabschnitte auf, von denen die Krümmung des ersten Krümmungsabschnitts, der den Scheitelbereich der Kontaktfläche ausbildet, geringer als die Krümmung eines an diesen ersten Krümmungsabschnitt anschließenden zweiten Krümmungsabschnitts ist, kann damit sowohl die Handhabung als auch die Messgenauigkeit der Vorrichtung deutlich verbessert werden. So kann zunächst der zweite Krümmungsabschnitt mit der größeren Krümmung dafür sorgen, dass selbst ein eventuell vorhandenes, dichtes Fell leichter durchdrungen wird, um die Elektroden standfest in Kontakt mit der Haut des Tiers zu bringen bzw. zu halten. Daher bedarf es auch keiner Verwendung eines leitfähigen Gels bzw. eines Abrasierens des Fells an den entsprechenden Stellen, was die Handhabung der Vorrichtung erheblich erleichtern kann.
Durch den ersten daran anschließenden Krümmungsabschnitt mit der im Vergleich zum zweiten Krümmungsabschnitt kleineren Krümmung kann in weiterer Folge sichergestellt werden, dass der vordere Bereich der Elektrode gegenüber dem zweiten Krümmungsabschnitt abgeflachter verläuft, sodass die ansonsten bei großer Krümmung bekannten Druckstellen und/oder Verletzungen am Tier ausbleiben können. Der Tragekomfort der erfindungsgemäßen Vorrichtung kann daher gegenüber dem Stand der Technik deutlich verbessert werden.
Zudem kann ein schmerzbedingter Tragestress vermieden werden, was das Auftreten verfälschter Messergebnisse reduziert. Die Standfestigkeit der Messdatenerfassung der Vorrichtung kann somit wesentlich erhöht werden. Erfindungsgemäß wird so eine einfach handzuhabende und zuverlässig genaue Ergebnisse liefernde Vorrichtung zur Messung der elektrischen Herzaktion eines Tieres zur Verfügung gestellt.

Im Allgemeinen wird festgehalten, dass unter elektrischer Herzaktion sowohl die Herzrate bzw. Herzfrequenz des Tieres als auch die Herzratenvariabilität (HRV) und weitere Parameter verstanden werden können, die aus der Herzkurve abgeleitet werden.

Im Allgemeinen wird weiter festgehalten, dass unter der Krümmung eines Krümmungsabschnitts dessen durchschnittliche Krümmung verstanden werden kann. So ist es vorstellbar, dass sich die Krümmung der Kontaktfläche beispielsweise kontinuierlich bis zum Scheitelbereich verringert. Eine derartige Elektrode könnte etwa einen elliptischen Querschnitt aufweisen.

Die Elektroden können konstruktiv einfach gestaltet werden, wenn die Krümmungsabschnitte je eine im Wesentlichen konstante Krümmung aufweisen. Dabei ist es vorstellbar, dass die unterschiedlichen Krümmungsabschnitte beispielsweise durch einzelne unterschiedliche Krümmungsradien beschrieben werden, wodurch sich eine einfach herzustellende Elektrode ergeben kann. Vorzugsweise lässt sich der Querschnitt der Elektrode, in Querrichtung des Gurts, in Kreise mit den entsprechenden Krümmungsradien einhüllen.

Weist die Kontaktfläche einen dritten Krümmungsabschnitt auf, in dem die Krümmungen der beiden aneinander anschließenden ersten und zweiten Krümmungsabschnitte fließend ineinander übergehen, kann eine eventuell scharfkantige Unstetigkeit im Übergang zwischen den beiden Krümmungsabschnitten vermieden werden. Der Tragekomfort der Vorrichtung kann sich damit weiter verbessern.

Der Kontaktsicherheit der Elektroden mit dem Tier kann weiter gesteigert werden, wenn die Elektroden in Längsrichtung des Gurtes eine konvexe Krümmung aufweisen. Damit ist nämlich erreichbar, dass die Elektroden in ihrem Durchdringungsvermögen durch das Fell weiter verbessert werden. Eine zuverlässig funktionierende Vorrichtung zur Messung der Herzaktion kann dadurch geschaffen werden.

Weisen die Elektroden eine radialsymmetrische Form auf, so kann der konstruktive Aufwand an der Vorrichtung reduziert werden. Insbesondere ist die Form der Elektroden als abgeflachter Kugelabschnitt darstellbar, wobei die Kugelradien der unterschiedlichen Krümmungsabschnitte ineinander übergehen. Außerdem kann diese Form einen besonders hohen Tragkomfort bieten und also eine zuverlässige und genaue Messung der Herzaktion eines Tieres ermöglichen.

Unter anderem kann die Signaldämpfung zwischen Elektroden und Messelektronik reduziert werden, wenn die Vorrichtung eine Messelektronik aufweist, die mit den Elektroden über elektrische Leitungen verbunden ist. Dies kann die Messgenauigkeit der Vorrichtung weiter erhöhen.

Die oben genannten Vorteile können weiter verbessert werden, wenn die elektrischen Leitungen im Gurt eingebettet sind. So lässt sich die Vorrichtung robuster gegenüber äußeren Einflüssen gestalten und kann Fehlfunktionen durch einwirkende mechanische oder chemische Einflüsse vorbeugen.

Verlaufen die elektrischen Leitungen mäanderförmig längs des Gurts, kann dies auch bei Zugbelastungen auf den Gurt eine sichere elektrische Verbindung gewährleisten. Die Standfestigkeit des Gurts kann dadurch erhöht werden.

Besteht der Gurt zumindest bereichsweise aus einem elastischen Material, so kann der Tragekomfort der Vorrichtung zusätzlich verbessert werden. Zudem können aufgrund der Elastizität Druckstellen vermieden und eine konstante Vorspannung auf die Elektroden ausgeübt werden, um den Kontakt der Elektroden zur Haut ebenfalls zuverlässiger auszubilden.

Ein einfaches Vorsehen der Vorrichtung an einem Tier kann ermöglicht werden, wenn der Gurt verschließbar und wieder öffenbar ausgebildet ist. So kann die Vorrichtung etwa um den Bauch des Tieres gelegt werden, und am Rücken verschlossen werden. Im Allgemeinen sind als Verschluss etwa Klettverschlüsse, Hakenverschlüsse, Schnallen oder Knöpfe vorstellbar.

Weist die Vorrichtung zudem eine Telematikeinrichtung zur Übertragung der Messdaten an einen Empfänger auf, so kann eine kabellose Verbindung zwischen Anzeige- bzw. Aufnahmegerät und Messelektronik der Vorrichtung geschaffen werden. Damit kann die Handhabung der Vorrichtung weiter verbessert und vereinfacht werden. Zudem sind Sicherheit und Zuverlässigkeit erhöhbar, da keine Kabelverbindungen über den Körper des Tieres gelegt werden müssen.

In den Figuren ist beispielsweise der Erfindungsgegenstand anhand eines Ausführungsbeispiels näher dargestellt. Es zeigen
Fig. 1 eine Querschnittsansicht durch die Vorrichtung in Längsrichtung,
Fig. 2 eine abgerissene Schnittansicht entlang II gemäß Fig. 1.

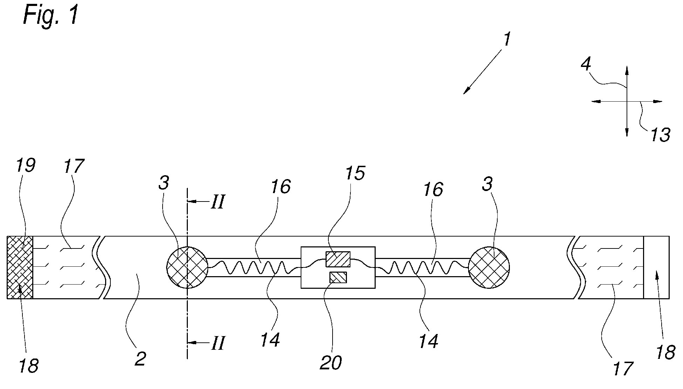

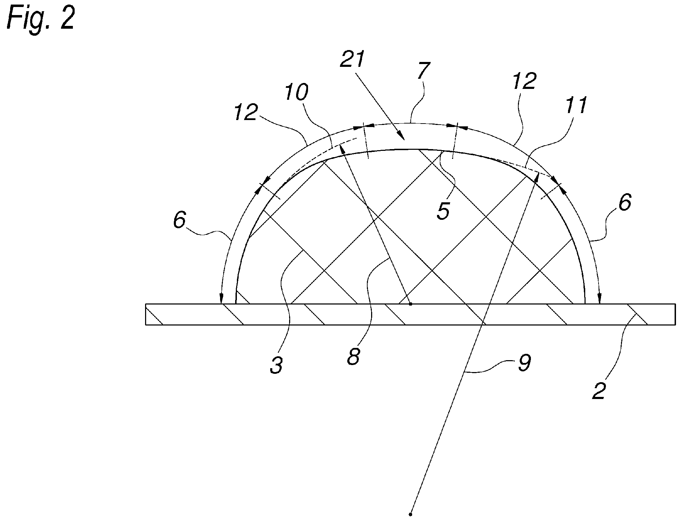

Gemäß Fig. 1 wird eine Vorrichtung 1 zur Messung der elektrischen Herzaktion eines nicht näher dargestellten Tieres gezeigt. Hierzu weist die Vorrichtung 1 einen Gurt 2 auf, an dem zwei elektrisch leitende Elektroden 3 vorgesehen sind. Diese Elektroden 3 bestehen aus einem gegen Körperschweiß resistenten Material, beispielsweise Edelstahl oder beschichtetes Metall etc., da diese mit der Haut eines Tieres in Kontakt gebracht werden müssen, um die Herzaktion bzw. die Herzkurve des Tieres messen zu können. Zur Herstellung des Kontakts weisen die Elektroden 3 in Querrichtung 4 des Gurtes 2 eine konvex gekrümmte Kontaktfläche 5 auf, wodurch die Elektrode 3 besonders leicht das Fell des Tiers zur Haut hin durchdringen kann. Die konvex gekrümmte Kontaktfläche 5 ist in der Fig. 2 insbesondere dargestellt.

Trotz der Funktion des erleichterten Durchdringens des Fells durch die Elektrode 3 kann das Tier vor einem aversiven Sinneserlebnis (Schmerzempfinden) geschützt bleiben, indem die erfindungsgemäße Kontaktfläche 5 mehrere Krümmungsabschnitte 6, 7 aufweist. Dabei ist die Krümmung des ersten Krümmungsabschnitts 7, der den Scheitelbereich der Kontaktfläche 5 ausbildet, geringer als die Krümmung eines an diesen ersten Krümmungsabschnitt 7 anschließenden zweiten Krümmungsabschnitts 6. Dadurch kann eine Abflachung 21 im Scheitelbereich an der Kontaktfläche 5 der Elektrode 3 ausgebildet werden, womit Druckstellen und/oder Verletzungen am Tier vermieden werden. Ein schmerzbedingter Tragestress tritt daher nicht ein, wodurch die Erfindung stets äußerst genaue Messergebnisse sicherstellen kann.

Wie im Ausführungsbeispiel dargestellt, können die einzelnen Krümmungsabschnitte 6, 7 dabei eine im Wesentlichen konstante Krümmung aufweisen. In diesem Fall können die Krümmungsabschnitte 6, 7 als Bereiche mit konstanten Krümmungsradien 8, 9 angesehen werden, wobei Krümmungsabschnitte 6, 7 der Kontaktfläche 5 jeweils in die zugehörigen Kreiskonturen 10, 11 der Krümmungsradien 8, 9 eingeschrieben werden können.

Um einen stufen- und kantenlosen Übergang zwischen den Krümmungsabschnitten 6, 7 zu erreichen, ist jeweils zwischen den aneinander anschließenden Krümmungsabschnitten 6, 7 mit konstanter Krümmung ein dritter Krümmungsabschnitt 12 angeordnet, in denen die Krümmungen der Krümmungsabschnitte 6, 7 fließend, also ohne Ausbildung von Kanten oder dergleichen, ineinander übergehen.

Wie in Fig. 1 zu erkennen, weisen die Elektroden 3 in Längsrichtung 13 des Gurtes 2 ebenso eine konvexe Krümmung auf. Insbesondere sind die Elektroden 3 radialsymmetrisch ausgeführt. Der Querschnitt der Elektroden 3 in Querrichtung 4 des Gurts 2 - wie in Fig. 2 gezeigt - entspricht demgemäß exakt dem Querschnitt der Elektroden 3 in Längsrichtung 13 des Gurts 2. Die in Fig. 2 gezeigten Kreiskonturen 10, 11 der Krümmungsabschnitte 6, 7 entsprechen also dreidimensionalen Kreisoberflächen. Eine derart geformte Elektrode 3 weist eine besonders vorteilhafte Durchdringungsfähigkeit des Tierfells auf und ermöglicht eine zuverlässige und genaue Messung der Herzaktion des Tiers. Es wird erwähnt, dass sich die erfindungsgemäße Vorrichtung insbesondere aber nicht ausschließlich zur Anwendung bei Pferden bewähren kann.

In Fig. 1 ist weiter zu erkennen, dass die Elektroden 3 über elektrische Leitungen 14 mit einer Messelektronik 15 verbunden sind. Die Leitungen 14 verlaufen in Längsrichtung des Gurts mäanderförmig, wodurch die Leitung 14 standfest Längsdehnungen des Gurts aufnehmen kann.

Die Messelektronik 15 ist vorteilhafterweise auf dem Gurt 2 vorgesehen, um lange Leitungen 14 zu vermeiden und damit etwa eine hohe Messgenauigkeit zu ermöglichen. Besonders vorteilhaft kann zudem sein, wenn die Leitungen 14 in dem Gurt 2, in einer Verkapselung 16 eingebettet sind (beispielsweise in einer Vergussmasse). Dadurch können die Leitungen 14 vor äußeren Einflüssen geschützt und die Messgenauigkeit weiter erhöht werden.

In Fig. 1 ist weiter angedeutet, dass der Gurt 2 abschnittsweise ein elastisches Material 17 aufweisen kann. Dadurch ist eine einfache Anpassung an den Tierkörper erreichbar. Zudem kann eine Überspannung des Gurts 2 und damit eine Druckstellenbildung beim Tier vermieden werden. An den Enden des Gurts 2 sind Verschlüsse 18 angebracht. Diese sind schematisch als Klettverschluss 19 dargestellt. Es kann sich bei den Verschlüssen 18 jedoch auch um Schnallen, Knöpfe oder Hakenverschlüsse handeln.

Zudem weist die Vorrichtung 1 auf dem Gurt 2 eine Telematikeinrichtung 20 auf, welche die von der Elektronik 15 aufgezeichneten Messdaten an einen nicht näher dargestellten Empfänger kabellos übertragen kann.

## Patentansprüche

1. Vorrichtung zur Messung der elektrischen Herzaktion eines Tieres, mit einem Gurt (2) und mit zumindest zwei am Gurt (2) vorgesehenen Elektroden (3), die je eine in Querrichtung (4) des Gurts (2) konvex gekrümmte Kontaktfläche (5) aufweisen, **dadurch gekennzeichnet, dass** die konvex gekrümmten Kontaktflächen (5) mehrere Krümmungsabschnitte (6, 7) aufweisen, von denen die Krümmung des ersten Krümmungsabschnitts (7), der den Scheitelbereich der Kontaktfläche (5) ausbildet, geringer als die Krümmung eines an diesen ersten Krümmungsabschnitt (7) anschließenden zweiten Krümmungsabschnitts (6) ist.

2. Vorrichtung nach Anspruch 1, **dadurch gekennzeichnet, dass** die Krümmungsabschnitte (6, 7) jeweils eine im Wesentlichen konstante Krümmung aufweisen.

3. Vorrichtung nach Anspruch 1 oder 2, **dadurch gekennzeichnet, dass** die Kontaktfläche (5) einen dritten Krümmungsabschnitt (12) aufweist, in dem die Krümmungen der beiden aneinander anschließenden ersten und zweiten Krümmungsabschnitte (6, 7) fließend ineinander übergehen.

4. Vorrichtung nach Anspruch 1, 2 oder 3, **dadurch gekennzeichnet, dass** die Elektroden (3) in Längsrichtung (13) des Gurtes (2) eine konvexe Krümmung aufweisen.

5. Vorrichtung nach einem der Ansprüche 1 bis 4, **dadurch gekennzeichnet, dass** die Elektroden (3) eine radialsymmetrische Form aufweisen.

6. Vorrichtung nach einem der Ansprüche 1 bis 5, **dadurch gekennzeichnet, dass** die Vorrichtung eine Messelektronik (15) aufweist, die mit den Elektroden (3) über elektrische Leitungen (14) verbunden ist.

7. Vorrichtung nach Anspruch 6, **dadurch gekennzeichnet, dass** die elektrischen Leitungen (14) im Gurt (2) eingebettet sind.

8. Vorrichtung nach Anspruch 6 oder 7, **dadurch gekennzeichnet, dass** die elektrischen Leitungen (14) mäanderförmig längs des Gurts (2) verlaufen.

9. Vorrichtung nach einem der Ansprüche 1 bis 8, **dadurch gekennzeichnet, dass** der Gurt (2) zumindest bereichsweise aus einem elastischen Material (17) besteht.

10. Vorrichtung nach einem der Ansprüche 1 bis 9, **dadurch gekennzeichnet, dass** der Gurt (2) verschließbar und wieder öffenbar ausgebildet ist.

11. Vorrichtung nach einem der Ansprüche 1 bis 10, **dadurch gekennzeichnet, dass** die Vorrichtung (1) eine Telematikeinrichtung (20) zur Übertragung der Messdaten an einen Empfänger aufweist.
